(19) Europäisches Patentamt
European Patent Office
Office européen des brevets

(11) EP 2 298 174 A1

(12) EUROPÄISCHE PATENTANMELDUNG

(43) Veröffentlichungstag:
23.03.2011 Patentblatt 2011/12

(51) Int Cl.:
$A61B\ 6/00$ (2006.01)

(21) Anmeldenummer: 10177442.0

(22) Anmeldetag: 17.09.2010

(84) Benannte Vertragsstaaten:
AL AT BE BG CH CY CZ DE DK EE ES FI FR GB GR HR HU IE IS IT LI LT LU LV MC MK MT NL NO PL PT RO SE SI SK SM TR
Benannte Erstreckungsstaaten:
BA ME RS

(30) Priorität: 17.09.2009 EP 09011848

(71) Anmelder: SIEMENS AKTIENGESELLSCHAFT
80333 München (DE)

(72) Erfinder: Navab, Nassir
81247, München (DE)

(54) Verfahren zum Erzeugen eines aus zumindest zwei Einzelröntgenbildern zusammengesetzten Röntgenbildes

(57) Unterzieht man eine Röntgenstrahlenquelle mit einem mit dieser gekoppelten Röntgenstrahlendetektor einer reinen Drehung, so lassen sich nacheinander in unterschiedlichen Drehstellungen aufgenommene Bilder zu einem Panoramabild zusammensetzen. Regelmäßig lässt sich dies jedoch nicht durchführen, sondern es ist stets ein translatorischer Anteil bei der Bewegung der Röntgenstrahlenquelle zu berücksichtigen. Bei der Erfindung wird die translatorische Bewegung der Röntgenstrahlenquelle (10, 10') dadurch genau ausgeglichen, dass die Lagervorrichtung für das abzubildende Objekt (14) genau die gleiche translatorische Bewegung erfährt.

EP 2 298 174 A1

**Beschreibung**

Die Erfindung betrifft ein Verfahren zum Erzeugen eines Röntgenbildes eines auf einer Lagervorrichtung befindlichen Objekts, typischerweise eines Patienten auf einem Patiententisch. Röntgenbildgebungseinrichtungen wie z. B. eine Röntgenangiographievorrichtung mit einem Röntgen-C-Bogen haben im Vergleich zur Größe eines typischen Menschen einen nicht übermäßig großen Sichtbereich, bilden also durch ein einziges Röntgenbild (Einzel-Röntgenbild) nur einen kleinen Teil eines Patienten ab. Für zahlreiche Untersuchungen und insbesondere auch während eines interventionellen Eingriffs am Patienten ist es wünschenswert, über Röntgenbilder zu verfügen, die einen wesentlich größeren Bereich als ein Einzel-Röntgenbild darstellen. Man behilft sich hierbei damit, eine Mehrzahl von Einzel-Röntgenbildern (zumindest zwei davon)aufzunehmen und diese unter Einsatz einer mathematischen Vorschrift zu einem Röntgenbild zusammenzusetzen.

Aus dem Gebiet der Optik ist es bekannt, Panoramabilder zu erzeugen. Hierbei wird eine optische Kamera um eine Achse geschwenkt. Panoramabilder sind präzise, ohne Parallaxen berechenbar, wenn die Drehung um den optischen Mittelpunkt erfolgt, also den Mittelpunkt (das Zentrum) des Raumwinkelsegments, das durch die Kamera abgebildet wird.

Möchte man flache Objekte mit einer Mehrzahl von Einzel-Bildern abbilden, ist das Erfordernis der Drehung um den optischen Mittelpunkt nicht ganz so streng, sondern es kann auch ein translatorischer Anteil in der Bewegung der Kamera gegeben sein, der herausrechenbar ist.

Bei der Röntgenbildgebung ist wegen des Aufbaus der zugehörigen Vorrichtung regelmäßig ein translatorischer Anteil dabei, was den Standort der Röntgenstrahlenquelle angeht. Die abzubildenden Objekte sind jedoch nicht flach. Aus diesem Grund ist nicht einfach aus einer Mehrzahl von Röntgenbildern ein zusammengesetztes Bild oder Panoramabild berechenbar. Stattdessen hat man mit dem Problem der Parallaxe zu kämpfen. Im Zusammenhang mit dem Zusammensetzen von Einzel-Röntgenbildern beschreiben die Autoren Z. Yaniv und L. Joskowicz in "Long bone panoramas from fluoroscopic x-ray images", IEEE Transactions on Medical Imaging 23(1) (2004) S. 26 bis 35 ein Verfahren zur Korrekturberechnung, um Effekte der Parallaxe auszurechnen. Der Artikel von L. Wang et al. "Long bone x-ray image stitching using a camera augmented mobile C-arm", in: MICCAI 2008, Volume 15242 of LNCS, Springer (2008), S. 578 bis 586 beschreibt ebenfalls eine derartige Parallaxenkorrektur.

Auch hier funktioniert das Verfahren jedoch nur dann ideal, wenn ein ebenes Objekt abgebildet wird. Gerade abzubildende Knochenstrukturen eines Patienten sind jedoch nicht eben.

Es ist daher Aufgabe der vorliegenden Erfindung, einen Weg aufzuzeigen, wie vermieden werden kann, dass aufgrund einer translatorischen Bewegung der Röntgenstrahlenquelle Parallaxeneffekte eine optimale Zusammensetzung ihrer Einzel-Röntgenbilder zu einem einzigen Röntgenbild verhindern.

Die Aufgabe wird durch ein Verfahren mit den Merkmalen gemäß Patentanspruch 1 gelöst.

Bei der Erfindung wird die Röntgenstrahlenquelle mit Röntgenstrahlendetektor zwischen jeder Aufnahme eines Einzelbildes gedreht, wobei das Ziel in einer Drehung um eine die Röntgenstrahlenquelle durchlaufende Achse besteht. Bei einer (regelmäßig auftretenden) zu besagter Drehung um eine die Röntgenstrahlenquelle durchlaufende Achse zusätzlichen translatorischen Bewegung der Röntgenstrahlenquelle zwischen der Aufnahme von Einzel-Röntgenbildern wird nun im Rahmen der Erfindung die gleiche translatorische Bewegung der Lagervorrichtung bewirkt (also mit dem selben Verschiebungsvektor). Zweck des Ganzen ist, dass die Relativlage zwischen der Röntgenstrahlenquelle und dem Objekt gleich bleibt. Wegen des Ausgleichs der translatorischen Bewegung der Röntgenstrahlenquelle durch die translatorische Bewegung der Lagervorrichtung spielt dann nur noch die beabsichtigte Drehung der Röntgenstrahlenquelle um eine die Röntgenstrahlenquelle durchlaufende Achse bei der weiteren Datenverarbeitung eine Rolle, die sodann erfolgt, um die Einzel-Röntgenbilder zusammenzusetzen. Hier kann dann an die bekannten Methoden aus der Optik angeknüpft werden. Es gibt insbesondere wegen der Kompensation der translatorischen Bewegung keine Probleme mehr mit der Parallaxe, und es kann ein ideales Panoramabild bereitgestellt werden.

Es ist möglich, dass die Röntgenstrahlenquelle und der Röntgenstrahlendetektor von einem Röntgen-C-Bogen getragen werden, der seinerseits durch einen ersten Roboter bewegt wird. Ein solcher Roboter wird von einer Steuereinrichtung angesteuert. Nun kann vorgesehen sein, dass die Lagervorrichtung durch einen von derselben Steuereinrichtung angesteuerten zweiten Roboter bewegt wird. In der Steuereinrichtung stehen dann Daten zur Verfügung, die mit der Lage des Röntgen-C-Bogens im Raum in Zusammenhang stehen: Entweder sind dies Daten, die unmittelbar die Steuerbefehle bewirken oder darstellen, aus denen sich die Lage des Röntgen-C-Bogens ergibt; oder die Steuereinrichtung errechnet ständig neu die Lagekoordinaten und hält die aktuellen Lagekoordinaten vor. In beiden Fällen ist es möglich, aufgrund der vorhandenen Daten Steuerbefehle für den zweiten Roboter zu erzeugen derart, dass genau eine Kompensation der translatorischen Bewegung der Röntgenstrahlenquelle erfolgt. In diesem Aspekt funktioniert das erfindungsgemäße Verfahren automatisch, insbesondere was die Kompensation angeht. Es ist daher besonders komfortabel für die Bedienpersonen. Die Verwendung der beiden Roboter hat auch den Vorteil, dass es besonders viele Freiheitsgrade für die Bewegung des Röntgen-C-Bogens im Raum gibt, was die Flexibilität bei der Aufnahme von (Einzel-)Röntgenbildern erhöht.

Alternativ ist es möglich, die translatorische Bewegung der Röntgenstrahlenquelle zu erfassen (also zu messen) und dann für eine entsprechende Korrektur zu sorgen. Zur Messung können an sich beliebige Sensoren oder sonstige Messeinrichtungen eingesetzt werden. Die Erfassung erfolgt jedoch bevorzugt anhand von Bildern einer Kamera, wobei diese besonders bevorzugt dieselbe Sicht auf das Objekt hat, wie die Röntgenstrahlenquelle. Mit Hilfe dieser Kamerabilder kann die translatorische Bewegung der Lagervorrichtung bewirkt werden. Insbesondere kann auch aus den Kamerabildern ein Verschiebungsvektor abgeleitet werden. Eine Kamera hat dann die selbe Sicht auf das Objekt wie die Röntgenstrahlenquelle, wenn der selbe Raumwinkel, in den die Röntgenstrahlenquelle Strahlung abgibt, der Raumwinkel ist, den die Kamera beobachtet, also in dem Lichtstrahlen von einem Objekt zur Kamera gelangen. Dies ist ganz einfach dadurch realisierbar, dass in den Strahlengang von der Röntgenstrahlenquelle ausgehenden Röntgenstrahlen ein Spiegel eingekoppelt wird, auf den die Kamera blickt.

Bei einer bevorzugten Ausführungsform der Variante mit Benutzung der Kamera werden Bilder der Kamera auf einem Bildschirm angezeigt, und die Lagevorrichtung ist manuell verfahrbar, damit eine Bedienperson anhand von Bildern der Kamera die translatorische Bewegung der Röntgenstrahlendquelle ausgleicht. Dies beruht auf der Erkenntnis, dass möglicherweise eine händische Korrektur am effizientesten ist; die Bedienperson kann sich hierbei z. B. an einem Muster orientieren, das an der Lagervorrichtung oder dem Objekt auf der Lagervorrichtung bereitgestellt ist (auf diesen liegt oder an diesen angebracht ist).

Bei einer zweiten Ausführungsform dieser Variante mit Kamera wird anhand von Bildern der Kamera auf einen bei Aufnahme des ersten Einzel-Röntgenbildes gegebene Relativstellung zwischen Röntgenstrahlenquelle und Lagervorrichtung geregelt. Auch hier kann ein vorbestimmtes Muster zur Orientierung an der Lagervorrichtung oder dem Objekt bereitgestellt sein, und aufgrund einer Mustererkennung kann dann die Regelung dahingehend erfolgen, dass das Muster stets einen bestimmten Bildbereich in den Bildern der Kamera mit bestimmter Orientierung einnimmt.

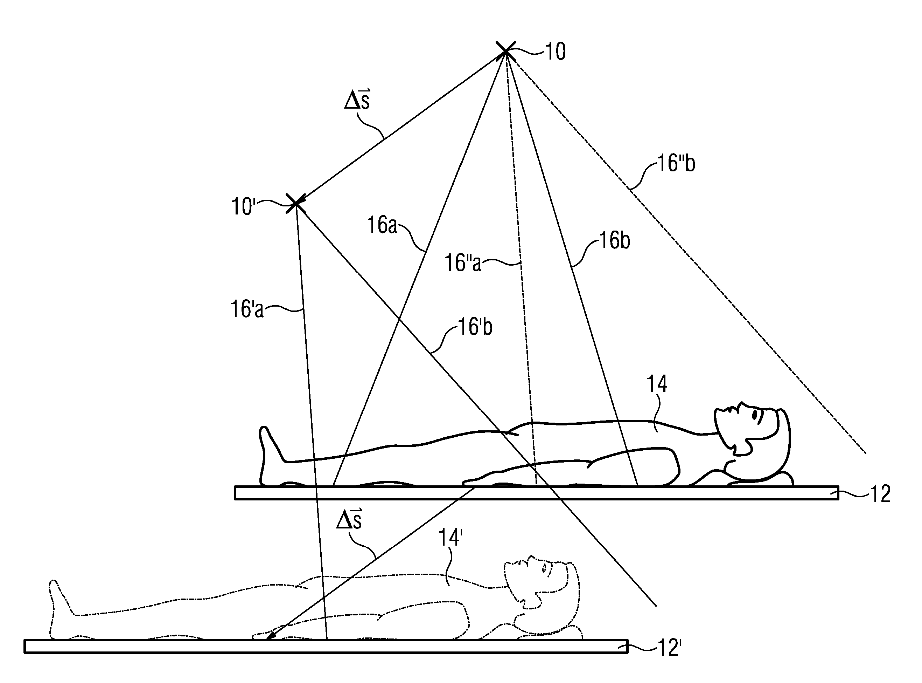

Nachfolgend wird eine bevorzugte Ausführungsform der Kamera unter Bezug auf die Zeichnung beschrieben, wobei FIG 1 schematisch eine Röntgenstrahlenquelle in zwei unterschiedlichen Stellungen und hierzu einen Patiententisch in entsprechenden Stellungen veranschaulicht.

Es soll mit Hilfe einer Röntgenstrahlenquelle 10 ein Röntgenbild eines auf einer Patientenlagervorrichtung (Patiententisch) 12 liegenden Patienten 14 gemacht werden. Die Röntgenstrahlenquelle 10 strahlt ein Strahlenbündel ab, das begrenzt ist, siehe die Bezugszeichen 16a und 16b für die begrenzten Röntgenstrahlen in FIG 1. Aus diesem Grunde ist lediglich ein Ausschnitt des Patienten 14 durch die Röntgenstrahlenquelle 10 abbildbar. Dreht man die Röntgenstrahlenquelle 10 um genau das Zentrum, von dem das Strahlenbündel ausgeht, so lassen sich nacheinander unterschiedliche Bereiche des Patienten 14 abbilden, und die so gewonnenen Einzel-Röntgenbilder lassen sich zu einem Gesamt-Röntgenbild zusammensetzen.

Nun lässt sich die Röntgenstrahlenquelle zusammen mit einem entsprechenden Röntgenstrahlendetektor (in der Figur nicht gezeigt) nicht immer ausschließlich einer reinen Drehung unterziehen; vielmehr kommt es regelmäßig beim Drehen der Röntgenstrahlenquelle 10 auch zu einer gleichzeitigen translatorischen Bewegung. Beispielsweise ist die Röntgenstrahlenquelle 10 zusammen mit dem zugehörigen Röntgenstrahlendetektor an einem Röntgen-C-Bogen angeordnet, der von einem Roboter bewegt wird. Dann sind an sich beliebige Lagen möglich, aber nicht in jeder Lage ist jede Bewegung möglich.

FIG 1 zeigt zusätzlich zu einer ersten Stellung der Röntgenstrahlenquelle 10 selbige in einer zweiten Stellung; dort ist sie mit der Bezugszahl 10' bezeichnet. Der Verschiebungsvektor ist mit $\vec{\Delta s}$ bezeichnet. Aufgrund der Drehung der Röntgenstrahlungsquelle wird das Strahlenbündel in eine andere Richtung abgegeben. In FIG 1 ist der Verschiebungsvektor $\vec{\Delta s}$ etwas übertrieben dargestellt.

Dann wird gemäß der Erfindung der Patiententisch 12 genau um denselben Verschiebungsvektor verschoben wie die Röntgenstrahlenquelle 10. Dadurch wird das erzielt, was durch die Drehung erreicht werden soll: Es werden nacheinander unterschiedliche Bereiche des Patienten 14 abgebildet. Es wird somit eine Relativlage geschaffen, die den Effekt hat, als sei die Röntgenstrahlenquelle 10 einer reinen Drehung unterzogen worden. Es wird also bei der Stellung der Röntgenstrahlenquelle gemäß Röntgenstrahlenquelle 10' mit dem Strahlenbündel, das durch die Strahlen 16'a und 16'b begrenzt ist, derselbe Effekt erzielt, als sei von der Röntgenstrahlenquelle 10 ein Strahlenbündel, das durch die Strahlen 16"a und 16"b begrenzt ist, ausgegangen.

In der Figur überlappen die Bereiche des Patienten, die nacheinander abgebildet werden. Die Drehung lässt sich jedoch insbesondere auch so gestalten, dass genau aneinander angrenzende Bereiche des Patienten 14 nacheinander abgebildet werden.

Bei einer reinen Drehung der Röntgenstrahlenquelle 10 lassen sich jedoch stets sämtliche Bildpunkte der einzelnen Strahlen von der Röntgenstrahlenquelle 10 zum Patienten 14 hin zuordnen, und aus den Einzel-Röntgenbildern kann rechnerisch sehr einfach ein Röntgenbild zusammengesetzt werden.

Man kann dies wie folgt tun: Es wird eine Videokamera mit der Röntgenstrahlenquelle derart gekoppelt, dass die Kamera genau in den Raumwinkel blickt, in den die Röntgenstrahlenquelle Röntgenstrahlen abgibt; eine solche Vorrichtung ist aus der US 6,229,873 B1 bekannt, aus der US 6,447,163 B1 und aus der US 6,473,489 B2 weiter bekannt, und diese Vorrichtung kann vorliegend benutzt werden. Erfasst werden die Bewegungen der Kamera, und nicht die der Röntgenstrahlenquelle, und zwar anhand der Bilder eines optischen Musters, das am Patiententisch 12 angebracht wird. Es soll nun eine Abbildungsvorschrift, eine sogenannte ebene Homographie angegeben werden, die ein Einzel-Röntgenbild auf ein nachfolgend aufgenommenes Röntgenbild abzubilden ist. Es sei $K$ eine 3x3-Matrix im reellen Raum, die intrinsische Matrix der Kamera, die z. B. wiedergibt, wie die Vergrößerung der Kamera ist, etc. R sei die Drehmatrix, die angibt, wie die Kamera gedreht wird. $\Delta\vec{s}$ sei der Verschiebungsvektor. $\vec{n}^T$ ist ein Normalenvektor, und $d$ sei der Abstand zum Ursprung einer vordefinierten Ebene zum Koordinatensystem. Die Homographie $H$ wird allgemein angegeben als

$$H = KRK^{-1} + \frac{1}{d} K \, \Delta \, \vec{s} \, \vec{n}^{T} \, K^{-1} \qquad (1).$$

Durch die Kompensation, die oben anhand von FIG 1 erläutert wird, also durch die Bewegung des Patiententischs genau um denselben Verschiebungsvektor $\Delta\vec{s}$ wie die Röntgenstrahlenquelle (wobei die Kompensation anhand von Bildern der Kamera erfolgt) wird $\Delta\vec{s}$ durch ein ($-\Delta\vec{s}$) genau ausgeglichen, der zweite Term entfällt somit in Gleichung (1).

Man erhält somit einfach $H = KRK^{-1}$.

Es muss so lediglich die Drehung der Kamera erfasst werden, und dann ist die Homographie ableitbar, sofern man die intrinsische Matrix der Kamera $K$ ebenfalls kennt.

Näheres zu der Berechnung eines Panoramabildes, also eines aus Einzel-Röntgenbildern zusammengesetzten Röntgenbildes, ist in dem eingangs zitierten Artikel von Z. Yaniv und L. Joskowicz beschrieben.

Es sei noch auf Folgendes hingewiesen: Bei manchen Anordnungen kann es vorteilhaft sein, wenn das Konzept einer virtuellen Detektorebene verwendet wird, um die gesuchte Homographie abzuleiten. Dieses Konzept ist im US-Patent 6,236,704 erläutert.

Durch die Erfindung erhält man Röntgenbilder, die aus einer Mehrzahl von Einzel-Röntgenbildern zusammengesetzt sind und daher z. B. vorliegend den gesamten Patienten wiedergeben, auch wenn das Srahlenbündel bei der Aufnahme eines Einzel-Röntgenbildes lediglich einen Teil des Patienten abbildet.

## Patentansprüche

1. Verfahren zum Erzeugen eines Röntgenbildes eines auf einer Lagervorrichtung befindlichen Objekts, wobei das Röntgenbild aus zumindest zwei Einzel-Röntgenbildern zusammengesetzt wird, die mittels einer Röntgenstrahlenquelle (10) und eines Röntgenstrahlendetektors gewonnen werden, wobei die Röntgenstrahlenquelle mit dem Röntgenstrahlendetektor zwischen jeder Aufnahme eines Einzel-Röntgenbildes gedreht wird, und wobei bei einer zusätzlichen translatorischen Bewegung der Röntgenstrahlenquelle zwischen der Aufnahme von Einzel-Röntgenbildern die gleiche translatorische Bewegung der Lagervorrichtung (12) bewirkt wird, damit die Relativlage zwischen der Röntgenstrahlenquelle (10) und dem Objekt (14) gleich bleibt, sodass bei einer Datenverarbeitung zum Zusammensetzen der Einzel-Röntgenbilder lediglich eine Drehung der Röntgenstrahlenquelle (10) um eine die Röntgenstrahlenquelle durchlaufende Achse zwischen der Aufnahme von Einzel-Röntgenbildern zu berücksichtigen ist.

2. Verfahren nach Anspruch 1,
**dadurch gekennzeichnet, dass** ein die Röntgenstrahlenquelle (10) und den Röntgenstrahlendetektor tragender Röntgen-C-Bogen durch einen von einer Steuereinrichtung angesteuerten ersten Roboter bewegt wird, und dass die Lagervorrichtung durch einen von derselben Steuereinrichtung angesteuerten zweiten Roboter bewegt wird, und dass anhand von in der Steuereinrichtung vorhandenen Daten, die mit der Lage des Röntgen-C-Bogens in Zusammenhang stehen, Steuerbefehle für den zweiten Roboter erzeugt werden.

3. Verfahren nach Anspruch 1,
**dadurch gekennzeichnet, dass** die translatorische Bewegung der Lagervorrichtung gemessen wird.

4. Verfahren nach Anspruch 3,
**dadurch gekennzeichnet, dass** anhand von Bildern einer Kamera, die bevorzugt dieselbe Sicht auf das Objekt hat wie die Röntgenstrahlenquelle, die translatorische Bewegung der Lagervorrichtung bewirkt wird.

5. Verfahren nach Anspruch 4, **dadurch gekennzeichnet, dass** Bilder der Kamera auf einem Bildschirm angezeigt werden und die Lagervorrichtung manuell verfahrbar ist, damit eine Bedienperson anhand von Bildern der Kamera die translatorische Bewegung der Röntgenstrahlenquelle ausgleicht.

6. Verfahren nach Anspruch 4, **dadurch gekennzeichnet, dass** anhand von Bildern der Kamera auf eine bei Aufnahme des ersten Einzel-Röntgenbildes gegebene Relativstellung zwischen Röntgenstrahlenquelle (10) und Lagervorrichtung (12) geregelt wird.

7. Verfahren nach einem der Ansprüche 3 bis 6, **dadurch gekennzeichnet, dass** ein vorbestimmtes Muster zur Orientierung an der Lagervorrichtung (12) oder dem Objekt (14) bereitgestellt wird.

8. Röntgenbildaufnahmevorrichtung mit einer Röntgenstrahlenquelle (10, 10') und einem Röntgenstrahlendetektor und einer Lagervorrichtung (12, 12') für in Röntgenbildern abzubildende Objekte, wobei sowohl die Einheit aus Röntgenstrahlenquelle (10, 10') und Röntgenstrahlendetektor einerseits und Lagervorrichtung (12, 12') andererseits im Raum beweglich sind, **dadurch gekennzeichnet, dass** die Röntgenbildaufnahmevorrichtung dazu ausgelegt ist, gemäß dem Verfahren nach einem der Ansprüche 1 bis 7 zu arbeiten.

9. Röntgenbildaufnahmevorrichtung nach Anspruch 8, mit einer Steuereinrichtung, die die Durchführung des erfindungsgemäßen Verfahrens zu bewirken ausgelegt ist.

Europäisches
Patentamt

European
Patent Office

Office européen
des brevets

**EUROPÄISCHER RECHERCHENBERICHT**

Nummer der Anmeldung

EP 10 17 7442

| | EINSCHLÄGIGE DOKUMENTE | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | WO 2008/020372 A2 (PHILIPS INTELLECTUAL PROPERTY [DE]; KONINKL PHILIPS ELECTRONICS NV [NL] 21. Februar 2008 (2008-02-21) * Zusammenfassung; Abbildung 2b * * Seite 1, Zeile 13 - Seite 2, Zeile 10 * * Seite 3, Zeile 25 - Seite 4, Zeile 16 * * Seite 5, Zeile 1 - Seite 6, Zeile 2 * * Seite 9, Zeile 30 - Seite 10, Zeile 19 * * Seite 12, Zeile 7 - Seite 13, Zeile 4 * ----- | 1-9 | INV. A61B6/00 |
| A | GB 2 232 332 A (GEN ELECTRIC [US]) 5. Dezember 1990 (1990-12-05) * Zusammenfassung; Abbildungen 2-9 * * Seite 4, Zeile 15 - Seite 7, Zeile 13 * ----- | 1-9 | |
| A | US 2004/156476 A1 (HALSMER MATTHEW AARON [US] ET AL) 12. August 2004 (2004-08-12) * Zusammenfassung; Abbildungen 2, 3 * * Absatz [0003] - Absatz [0004] * ----- | 1-9 | |

RECHERCHIERTE
SACHGEBIETE (IPC)

A61B

Der vorliegende Recherchenbericht wurde für alle Patentansprüche erstellt

| Recherchenort | Abschlußdatum der Recherche | Prüfer |
|---|---|---|
| München | 30. November 2010 | Sonntag, Anne |

KATEGORIE DER GENANNTEN DOKUMENTE

X : von besonderer Bedeutung allein betrachtet
Y : von besonderer Bedeutung in Verbindung mit einer
   anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
   nach dem Anmeldedatum veröffentlicht worden ist
D : in der Anmeldung angeführtes Dokument
L : aus anderen Gründen angeführtes Dokument
.......................................................................................................
& : Mitglied der gleichen Patentfamilie, übereinstimmendes
   Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**                    EP 10 17 7442

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

30-11-2010

| Im Recherchenbericht angeführtes Patentdokument | Datum der Veröffentlichung | Mitglied(er) der Patentfamilie | Datum der Veröffentlichung |
|---|---|---|---|
| WO 2008020372 A2 | 21-02-2008 | CN 101500488 A<br>EP 2053971 A2<br>JP 2010500146 T<br>US 2010172472 A1 | 05-08-2009<br>06-05-2009<br>07-01-2010<br>08-07-2010 |
| GB 2232332 A | 05-12-1990 | AU 622307 B2<br>AU 5304890 A<br>DE 4016245 A1<br>FR 2650684 A1<br>IT 1248819 B<br>JP 3037051 A<br>TR 27870 A<br>US 5032990 A | 02-04-1992<br>06-12-1990<br>13-12-1990<br>08-02-1991<br>30-01-1995<br>18-02-1991<br>11-10-1995<br>16-07-1991 |
| US 2004156476 A1 | 12-08-2004 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- US 6229873 B1
- US 6447163 B1
- US 6473489 B2
- US 6236704 B

### In der Beschreibung aufgeführte Nicht-Patentliteratur

- **Z. Yaniv ; L. Joskowicz.** Long bone panoramas from fluoroscopic x-ray images. *IEEE Transactions on Medical Imaging,* 2004, vol. 23 (1), 26-35
- Long bone x-ray image stitching using a camera augmented mobile C-arm. **von L. Wang et al.** MICCAI 2008. Springer, 2008, vol. 15242, 578-586